# NEUE EUROPÄISCHE PATENTSCHRIFT

(11) **EP 1 663 921 B2**
(45) Veröffentlichungstag und Bekanntmachung des Hinweises auf die Entscheidung über den Einspruch: **25.08.2010**
(45) Hinweis auf die Patenterteilung: 02.05.2007
(21) Anmeldenummer: 04764917.3
(22) Anmeldetag: 07.09.2004
(51) Int. Cl.: C07C 17/16

(54) **VERFAHREN ZUR HERSTELLUNG VON HALOGENALKANEN AUS ALKOHOLEN**
METHOD FOR PRODUCING HALOALKANES FROM ALCOHOLS
PROCEDE DE PRODUCTION D'ALCANES HALOGENES A PARTIR D'ALCOOLS

(30) Priorität: 08.09.2003 DE 10341308
(43) Veröffentlichungstag der Anmeldung: 07.06.2006
(73) Patentinhaber: BASF SE, 67056 Ludwigshafen (DE)
(72) Erfinder: STEGMANN, Veit, 68165 Mannheim (DE); MASSONNE, Klemens, 67098 Bad Dürkheim (DE)
(74) Vertreter: Ellwanger, Arndt
(86) Internationale Anmeldenummer: PCT/EP2004/009974
(87) Internationale Veröffentlichungsnummer: WO 2005/026089

(56) Entgegenhaltungen:
- EP-A- 0 428 166
- CH-A5- 589 015
- JP-A- 56 150 026
- JP-B- 4 934 646
- US-A- 3 607 953
- US-A- 4 115 390
- US-A- 5 233 108
- US-A- 5 723 704
- US-A- 5 767 330
- REN, R.X. ET AL: "Mild conversion of alcohols to alkyl halides using halide-based ionic liquids at room temperature" ORGANIC LETTERS, Bd. 3, Nr. 23, 2001, Seiten 3727-3728, XP002319406 in der Anmeldung erwähnt
- DATABASE PRODUKTINFORMATION FIRMA SIGMA-ALDRICH 'Pyridine hydrochloride'
- GREEN CHEMISTRY Bd. 5, 2003, Seiten 303 - 305
- 'Organikum', 2001, WILEY-VCH, WEINHEIM Seiten 226 - 227

## Beschreibung

Die Erfindung betrifft ein Verfahren zur Herstellung von Halogenalkanen durch Umsetzung von Alkoholen mit Halogenwasserstoff in Gegenwart einer ionischen Flüssigkeit.

Das Verfahren entspricht der nachfolgenden allgemeinen Reaktionsgleichung, wobei R für eine beliebigen Alkylrest steht

R-OH + HX → R-X + H₂O.

In der Literatur sind bereits eine Vielzahl von Verfahren zur Halogenierung von Alkoholen mit Halogenwasserstoffen beschrieben. In diesen Verfahren wird die Reaktion in Gegenwart einer wässrigen Base (zumeist Alkylamine oder Pyridinderivate sowie deren Salze) durchgeführt, wobei die Base als Katalysator fungiert.

EP-A 0 428 166 beschreibt ein Verfahren zur Herstellung von Halogenalkanen mit 1 bis 4 Kohlenwasserstoffatomen in Gegenwart von Aminhydrohalogenid. Als dem Hydrohalogenid zugrundeliegende Amine werden beispielsweise Aniline, Pyridine, Chinoline, Phenylendiamine, α- und β-Naphthylamine oder Imidazole verwendet. Die Aminhydrohalogenide werden als wässrige Lösung eingesetzt, wobei ein 1,9 bis 11,9-fach molarer Überschuss an Wasser gegenüber dem Amin(hydrochlorid) eingesetzt wird.

EP-A 0 789 013 betrifft ein Verfahren zur Herstellung von Alkylchloriden mit 6 bis 16 C-Atomen durch Umsetzung der entsprechenden Alkohole mit Chlorwasserstoff in Gegenwart einer nicht näher spezifizierten wässrigen Alkylpyridinhydrochlorid-Lösung, wobei die Reaktion bei einer Temperatur unterhalb der Siedetemperatur des Alkylchlorids durchgeführt und das gebildete Alkylchlorid mit Hilfe von zusätzlich eingespeister konzentrierter Salzsäure über Kopf abdestilliert wird.

Ein Verfahren zur Herstellung von tertiären Alkylchloriden aus den entsprechenden Alkoholen wird in US 3,852,368 beschrieben. Die Reaktion wird in Gegenwart eines organischen Lösungsmittels wie Heptan oder Benzol sowie der wässrigen Lösung eines Amins durchgeführt. Als Amine werden Tributylamin, Triethylamin, n-Butylamin oder Pyridin verwendet und Wasser mindestens in einem 1,4fach molaren Überschuss gegenüber dem Amin eingesetzt.

DE-A 199 26 165 betrifft ein Verfahren zur Herstellung von 1 ,3-Dichlorpropan durch Umsetzung von Bis(3-hydroxypropyl)ether mit Chlorwasserstoff in Gegenwart von tertiären basischen Stickstoffverbindungen oder anderen tertiären aliphatischen Basen als Katalysatoren. Geeignete tertiäre basische Stickstoffverbindungen sind Pyridin, Alkylpyridin, Chinolin oder Trialkylamin, wobei die tertiären basischen Stickstoffverbindungen in einem Gemisch mit Wasser vorliegen und das Verhältnis Base zu Wasser 0,87 bis 1,18 : 1 (Mol) beträgt.

DE-A 214 98 22 betrifft ein kontinuierliches Verfahren zur Herstellung von in 1,4- oder 1,5-Stellung chlorierten Kohlenwasserstoffen durch Umsetzung eines flüssigen Reaktionsgemisches aus 1,4- oder 1,5-Diolen und/oder entsprechenden cyclischen Ethern und Chlorwasserstoff. Die Reaktion wird in Gegenwart eines Katalysators (Tributylaminhydrochlorid oder N,N-Dimethylaminhydrochlorid) sowie von Wasser durchgeführt, wobei die Menge an eingesetztem Wasser 31 Mol%, bezogen auf die Menge an Katalysator, nicht unterschreitet.

R.X. Ren et al., Organic Letters, Band 3 (2001), 3727 - 3728 beschreibt die Umsetzung von Alkoholen in Gegenwart ionischer Flüssigkeiten (1-n-Butyl-3-methyl-imidazoliumhalogenide) sowie von Brönsted-Säuren bei Raumtemperatur. Für die in wässriger Form eingesetzten Brönsted-Säure HCl wird berichtet, dass über einen Zeitraum von> 48 Stunden keine Reaktion mit n-Butylalkohol in Gegenwart der ionischen Flüssigkeit (als Chlorid) eintritt.

Weiterhin werden in der Literatur auch Verfahren zur Herstellung von Halogenalkanen beschrieben, wobei der entsprechende Alkohol mit Halogenwasserstoff ohne Zugabe von zusätzlichem Wasser umgesetzt wird.

JP-A 2002179600 beschreibt ein Verfahren zur Herstellung von hochreinem 3-Chlor-1-propanol aus 1,3-Propandiol und gasförmigem Chlorwasserstoff in Gegenwart eines Katalysators. Als Katalysatoren werden insbesondere Zeolithe verwendet, es können jedoch auch quaternäre Ammoniumsalze wie Tetrabutylammonium- und Benzyltrimethylammoniumchlorid, Ammoniumsalze mit Octyl- oder Octadecylresten sowie Phosphoniumsalze eingesetzt werden. Um die Reaktion selektiv durchführen zu können, darf die Temperatur von 100 °C nicht überschritten werden.

JP-A 2001288127 betrifft ein Verfahren zur Herstellung von Alkylchloriden aus Alkohol und gasförmigem Chlorwasserstoff in Gegenwart von C₆₋₂₀-Alkyl-dimethylaminen als Katalysator. Die Reaktion wird bei 130 °C durchgeführt, der Gehalt an Katalysator beträgt 20 Mol% bezogen auf die Menge an verwendetem Alkohol. Im Rahmen eines Vergleichsversuchs wird Octyltrimethylammoniumchlorid als Katalysator verwendet. Die Verwendung dieses Katalysators wird in JP-A 2001288127 jedoch als ungünstig beschrieben, weil die Reaktion langsamer abläuft und im Verhältnis zum eingesetzten Alkohol äquimolare Mengen an Katalysator notwendig sind. Folglich trifft die Verwendung von Octyltrimethylammoniumchlorid nicht den Kern der Offenbarung von JP-A 2001288127, weil von dessen Verwendung abgeraten wird.

Die der Erfindung zugrundeliegende Aufgabe besteht in der Bereitstellung eines Verfahrens zur Herstellung von Halogenalkanen, das gegenüber den aus dem Stand der Technik bekannten Verfahren verbessert ist. Verbesserungen sollen hinsichtlich der Raum/Zeit-Ausbeute, der Ausbeute beziehungsweise Reinheit des Produkts und/oder des Umsatzes erzielt werden. Erfindungsgemäß wird diese Aufgabe durch ein Verfahren zur Herstellung von Halogenalkanen durch Umsetzung von Alkohol mit Halogenwasserstoff, **dadurch gekennzeichnet, dass** die Reaktion des Alkohols mit dem Halogenwasserstoff in Gegenwart einer ionischen Flüssigkeit zumindest zeitweise bei einer Temperatur von über 100°C durchgeführt wird und zumindest zum Zeitpunkt des Reaktionsbeginns der Wassergehalt maximal 25 Mol% bezogen auf die Menge an ionischer Flüssigkeit beträgt, wobei die ionische Flüssigkeit nicht Octyltrimethylammoniumchlorid ist und
das Kation der ionischen Flüssigkeit ein Imidazoliumion der Formel (e) 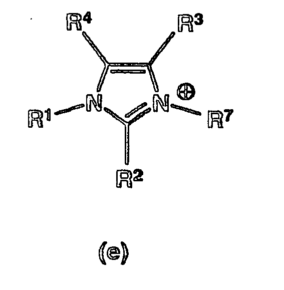 ist, worin R¹, R², R³, R⁴ und R⁷ unabhängig voneinander jeweils Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₆-C₁₂-Arvl, C₅-C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen und/oder Heterocyclen substituiert sein können, und
R⁷ kann darüber hinaus C₁-C₁₈-Alkyloyl (Alkylcarbonyl), C₁C₁₈-Alkyloxycarbonyl, C₅-C₁₂-Cycloalkylcarbonyl oder C₆-C₁₂-Aryloyl (Arylcarbonyl) bedeuten, wobei die genannten Reste jeweils durch funktionelle Gruppen, Aryl, Alkyl, Aryloxy, Alkyloxy, Halogen und/oder Heterocyclen substituiert sein können, wobei funktionelle Gruppen Carboxy, Carboxamid, Hydroxy, Amino, C₁-C₄-Alkylamino, Di-(C₁-C₄-alkyl)-amino, C₁-C₄- Alkyloxy carbonyl, Cyano oder C₁-C₄- Alkyloxy bedeuten.

Gegenüber Verfahren, die auf wässrigen Lösungen von ionischen Flüssigkeiten beziehungsweise den ionischen Flüssigkeiten entsprechenden Basen beruhen, hat das erfindungsgemäße Verfahren den Vorteil, dass dadurch eine Verbesserung der Raum/Zeit-Ausbeute, also eine beschleunigte Umsetzung von Alkoholen mit Halogenwasserstoffen, erzielt werden kann. Weiterhin ist im erfindungsgemäßen Verfahren auch ein verbesserter Umsatz gegenüber dem Verfahren unter Verwendung von ionischen Flüssigkeiten in wässriger Lösung festzustellen. Auch gegenüber den bereits bekannten Verfahren, die ohne Zugabe von zusätzlichem Wasser durchgeführt werden, weist das erfindungsgemäße Verfahren Vorteile auf. Die Verwendung von ionischen Flüssigkeiten anstelle der entsprechenden Basen hat den Vorteil, dass das Produkt (Halogenalkan) mit höherer Selektivität hergestellt werden kann, eine Erhöhung der Reaktionstemperatur bewirkt zudem einen deutlich verbesserten Umsatz.

Durch die Optimierung des Verfahrens zur Herstellung von Halogenalkanen, d.h. die Verwendung von ionischen Flüssigkeiten bei erhöhter Reaktionstemperatur und - zumindest anfänglicher - Wasserfreiheit beziehungsweise weitgehender Wasserfreiheit kann somit eine deutliche Verkürzung der Reaktionszeit der Alkohole mit Halogenwasserstoff, ein erhöhter Umsatz sowie die verbesserte Produktreinheit erzielt werden, was eine Kostenminimierung, insbesondere hinsichtlich größerer beziehungsweise großtechnischer Ansätze zur Folge hat.

Ein weiterer Vorteil des erfindungsgemäßen Verfahrens zeigt sich bei den Ausführungsformen, bei denen das bei der Reaktion freiwerdende Wasser kontinuierlich aus dem System entfernt wird, darin, dass Halogenwasserstoffe - insbesondere HCl - in wasserarmen Systemen weniger korrosiv ist, so dass die Apparatur, besonders der Reaktionskessel, geschont wird. Dadurch lassen sich der Aufwand und die Kosten für Wartung und Reparatur der Anlagen reduzieren.

Unter ionischen Flüssigkeiten, die auch flüssige Salze genannt werden, versteht man allgemein Salzschmelzen, deren Schmelzpunkt gewöhnlich unter 100 °C liegt (lonic Liquids in Synthesis von P. Wasserscheid und T. Welton (Herausgeber), 2003, S. 41-43, Viley-VCH Verlag, Weinheim (Deutschland)). Dabei ist festzuhalten, dass die Festsetzung des Schmelzpunktes auf 100 °C einen willkürlichen Grenzwert darstellt, da auch Verbindungen bekannt sind, die einen höheren Schmelzpunkt als 100°C aufweisen und trotzdem als ionische Flüssigkeiten eingesetzt werden können.

Im erfindungsgemäßen Verfahren werden als ionische Flüssigkeiten solche Verbindungen verstanden, die mindestens eine positive und mindestens eine negative Ladung aufweisen, insgesamt jedoch ladungsneutral sind, und einen Schmelzpunkt unter 200 °C aufweisen, bevorzugt unter 150 °C, besonders bevorzugt unter 100 °C. Im Rahmen der vorliegenden Erfindung ist Octyltrimethylammoniumchlorid von der ionischen Flüssigkeit ausgenommen. Im Rahmen der vorliegenden Erfindung weist die ionische Flüssigkeit als Kation ein Imidazoliumion gemäß der nachstehenden Formel (e) auf.

Die ionischen Flüssigkeiten können auch mehrere positive oder negative Ladungen aufweisen, beispielsweise 1 bis 5, bevorzugt 1 bis 4, besonders bevorzugt 1 bis 3, ganz besonders bevorzugt 1 bis 2, insbesondere jedoch je eine positive und negative Ladung.

Die Ladungen können sich an verschiedenen lokalisierten oder delokalisierten Bereichen innerhalb eines Moleküls befinden, also betainartig, oder auf je ein getrenntes Anion und Kation verteilt sein. Bevorzugt sind solche ionischen Flüssigkeiten, die aus mindestens einem Kation und mindestens einem Anion aufgebaut sind. Kation und Anion können, wie oben ausgeführt, ein oder mehrfach, bevorzugt einfach geladen sein. Als Anion und als Kation einer ionischen Flüssigkeit sind prinzipiell alle Anionen beziehungsweise Kationen denkbar.

Selbstverständlich sind auch Gemische verschiedener ionischer Flüssigkeiten oder Gemische von ionischen Flüssigkeiten mit Metallsalzen wie AlCl₃, FeCl₃, ZnCl₂ oder CoCl₃ denkbar.

Bevorzugte ionische Flüssigkeiten sind solche, die ein Molgewicht unter 1000 g/mol aufweisen, besonders bevorzugt unter 350 g/mol.

Bevorzugte ionische Flüssigkeiten weisen je eines der nachfolgend aufgeführten Anionen und Kationen auf. Dabei sind sämtliche Kombinationen von Anionen und Kationen mit umfasst, auch solche, bei denen Anion und Kation von unterschiedlicher Gewichtung sind, bspw. die Kombination eines noch mehr bevorzugten Kations mit einem mehr bevorzugten Anion.

Das Kation ist ausgewählt aus den Verbindungen der Formeln (e), 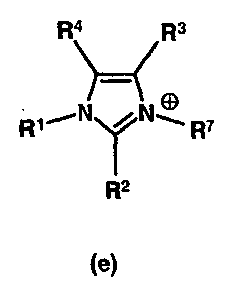 worin

R¹, R², R³, R⁴, und R⁷ unabhängig voneinander jeweils Wasserstoff, C₁ - C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl, C₆ - C₁₂-Aryl, C₅ - C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen und/oder Heterocyclen substituiert sein können. Die genannten Reste können unabhängig voneinander gegebenenfalls einen oder mehrere dieser Substituenten aufweisen.

R⁷ kann darüberhinaus C₁ - C₁₈-Alkyloyl (Alkylcarbonyl), C₁ - C₁₈- Alkyloxycarbonyl, C₅ - C₁₂-Cycloalkylcarbonyl oder C₆ - C₁₂-Aryloyl (Arylcarbonyl) bedeuten, wobei die genannten Reste jeweils durch funktionelle Gruppen, Aryl, Alkyl, Aryloxy, Alkyloxy, Halogen und/oder Heterocyclen substituiert sein können. Die genannten Reste können unabhängig voneinander gegebenenfalls einen oder mehrere dieser Substituenten aufweisen. In den Resten R¹ bis R⁴ und R³ bedeutet funktionelle Gruppe: Carboxy, Carboxamid, Hydroxy, Amino, C₁-C₄-Alkylamino, Di-(C₁-C₄-alkyl)-amino, C₁-C₄-Alkyloxycarbonyl, Cyano oder C₁-C₄-Alkyloxy.

Sofern einer oder mehrere der Reste R¹ bis R⁴ und R⁷ ein Alkylfragment aufweisen, ist dieses jeweils bevorzugt ein C₁-C₈-Alkyifragment, das sowohl unsubstituiert sein kann als auch einen oder mehrere der vorstehenden Substituenten aufweisen kann. Hierbei können die Reste R¹ bis R⁴ und R⁷ gleiche oder unterschiedliche Alkylfragmente, insbesondere gleiche oder unterschiedliche C₁-C₈-Alkylfragmente, aufweisen.

Sofern die vorstehend aufgeführten Kationen gemäß Formel (e) über ein oder mehrere (weitere) freie Elektronenpaare verfügen, beispielsweise im Heterocyclus und/oder den Resten R¹ bis R⁴ und R⁷, so sind auch diejenigen Formen dieser Kationen in der vorliegenden Erfindung mit umfasst, bei denen beispielsweise durch Einleiten von Halogenwasserstoff ein oder mehrere dieser freien Elektronenpaare der Kationen gegebenenfalls zusätzlich protoniert sind.

### Darin bedeuten

Alkyl: gegebenenfalls durch funktionelle Gruppen, Aryl, Alkyl, Aryloxy, Alkyloxy, Halogen und/oder Heterocyclen substituiertes C₁ - C₁₈-Alkyl, beispielsweise Methyl, Ethyl, Propyl, Isopropyl, n-Butyl, sec-Butyl, tert.-Butyl, Pentyl, Hexyl, Heptyl, Octyl, 2-Etylhexyl, 2,4,4-Trimethylpentyl, Decyl, Dodecyl, Tetradecyl, Heptadecyl, Octadecyl, 1,1-Dimethylpropyl, 1,1-Dimethylbutyl, 1,1,3,3-Tetramethylbutyl, Benzyl, 1-Phenylethyl, 2-Phenylethyl, α,α-Dimethylbenzyl, Benzhydryl, p-Tolylmethyl,1-(p-Butylphenyl)-ethyl, p-Chlorbenzyl, 2,4-Dichlorbenzyl, p-Methoxybenzyl, m-Ethoxybenzyl, 2-Cyanoethyl, 2-Cyanopropyl, 2-Methoxycarbonylethyl, 2-Ethoxycarbonylethyl, 2-Butoxycarbonylpropyl, 1,2-Di-(methoxycarbonyl)-ethyl, 2-Methoxyethyl, 2-Ethoxyethyl, 2-Butoxyethyl, Di-ethoxymethyl, Diethoxyethyl, 1,3-Dioxolan-2-yl, 1,3-Dioxan-2-yl, 2-Methyl-1,3-dioxolan-2-yl, 4-Methyl-1,3-dioxolan-2-yl, 2-Isopropoxyethyl, 2-Butoxypropyl, 2-Octyloxyethyl, Chlormethyl, 2-Chlorethyl, Trichlormethyl, Trifluormethyl, 1,1-Dimethyl-2-chlorethyl, 2-Methoxyisopropyl, 2-Ethoxyethyl, 2,2,2-Trifluorethyl, 2-Hydroxyethyl, 2-Hydroxypropyl, 3-Hydroxypropyl, 4-Hydroxybutyl, 6-Hydroxyhexyl, 2-Aminoethyl, 2-Aminopropyl, 3-Aminopropyl, 4-Aminobutyl, 6-Aminohexyl, 2-Methylaminoethyl, 2-Methylaminopropyl, 3-Methyl-aminopropyl, 4-Methylaminobutyl, 6-Methylaminohexyl, 2-Dimethylaminoethyl, 2-Dimethylaminopropyl, 3-Dimethylaminopropyl, 4-Dimethylaminobutyl, 6-Dimethylaminohexyl, 2-Hydroxy-2,2-dimethylethyl, 2-Phenoxyethyl, 2-Phenoxypropyl, 3-Phenoxypropyl, 4-Phenoxybutyl, 6-Phenoxyhexyl, 2-Methoxyethyl, 2-Methoxypropyl, 3-Methoxypropyl, 4-Methoxybutyl, 6-Methoxyhexyl, 2-Ethoxyethyl, 2-Ethoxypropyl, 3-Ethoxypropyl, 4-Ethoxybutyl oder 6-Ethoxyhexyl und,
gegebenenfalls durch ein oder mehrere Sauerstoff- und/oder Schwefelatome und/oder ein oder mehrere substituierte oder unsubstituierte Iminogruppen unterbrochenes C₂ - C₁₈-Alkyl beispielsweise 5-Hydroxy-3-oxa-pentyl, 8-Hydroxy-3,6-dioxa-octyl, 11-Hydroxy-3,6,9-trioxa-undecyl, 7-Hydroxy-4-oxa-heptyl, 11-Hydroxy-4,8-dioxa-undecyl, 15-Hydroxy-4,8,12-trioxa-pentadecyl, 9-Hydroxy-5-oxa-nonyl, 14-Hydroxy-5,10-oxa-tetradecyl, 5-Methoxy-3-oxa-pentyl, 8-Methoxy-3,6-dioxa-octyl, 11-Methoxy-3,6,9-trioxa-undecyl, 7-Methoxy-4-oxa-heptyl, 11-Methoxy-4,8-dioxa-undecyl, 15-Methoxy-4,8,12-trioxa-pentadecyl, 9-Methoxy-5-oxa-nonyl, 14-Methoxy-5,10-oxa-tetradecyl, 5-Ethoxy-3-oxa-pentyl, 8-Ethoxy-3,6-dioxa-octyl, 11-Ethoxy-3,6,9-trioxa-undecyl, 7-Ethoxy-4-oxa-heptyl, 11-Ethoxy-4,8-dioxa-undecyl, 15-Ethoxy-4,8,12-trioxa-pentadecyl, 9-Ethoxy-5-oxa-nonyl, 14-Ethoxy-5,10-oxa-tetradecyl, Butylthiomethyl, 2-Dodecylthioethyl oder 2-Phenyl-thioethyl.

Bilden zwei Reste einen Ring, so können diese Reste gemeinsam bedeuten 1,3-Propylen, 1,4-Butylen, 2-Oxa-1,3-propylen, 1-Oxa-1,3-propylen, 2-Oxa-1,3-propylen, 1-Oxa-1,3-propenylen, 1-Aza-1,3-propenylen, 1-C₁-C₄-Alkyl-1-aza-1,3-propenylen, 1,4-Buta-1,3-dienylen, 1-Aza-1,4-buta-1,3-dienylen oder 2-Aza-1,4-buta-1,3-dienylen.

Die Anzahl der Sauerstoff- und/oder Schwefelatome und/oder Iminogruppen ist nicht beschränkt. In der Regel beträgt sie nicht mehr als 5 in dem Rest, bevorzugt nicht mehr als 4 und ganz besonders bevorzugt nicht mehr als 3.

Weiterhin befindet sich zwischen zwei Heteroatomen (S, N, O) in der Regel mindestens ein Kohlenstoffatom, bevorzugt mindestens zwei.

Substituierte und unsubstituierte Iminogruppen können beispielsweise lmino-, Methylimino-, iso-Propylimino, n-Butylimino oder tert-Butylimino sein.

Weiterhin bedeuten

Aryl: gegebenenfalls durch funktionelle Gruppen, Aryl, Alkyl, Aryloxy, Alkyloxy, Halogen und/oder Heterocyclen substituiertes C₆ - C₁₂-Aryl, beispielsweise Phenyl, Tolyl, Xylyl, α-Naphthyl, β-Naphthyl, 4-Diphenylyl, Chlorphenyl, Dichlorphenyl, Trichlorphenyl, Difluorphenyl, Methylphenyl, Dimethylphenyl, Trimethylphenyl, Ethylphenyl, Diethylphenyl, iso-Propylphenyl, tert.-Butylphenyl, Dodecylphenyl, Methoxyphenyl, Dimethoxyphenyl, Ethoxyphenyl, Hexyloxyphenyl, Methylnaphthyl, Isopropylnaphthyl, Chlornaphthyl, Ethoxynaphthyl, 2,6-Dimethylphenyl, 2,4,6-Trimethylphenyl, 2,6-Diethoxyphenyl, 2,6-Dichlorphenyl, 4-Bromphenyl, 2- oder 4-Nitrophenyl, 2,4- oder 2,6-Dinitrophenyl, 4-Dimethylaminophenyl, 4-Acetylphenyl, Methoxyethylphenyl oder Ethoxyethylphenyl,

Cycloalkyl: gegebenenfalls durch funktionelle Gruppen, Aryl, Alkyl, Aryloxy, Alkyloxy, Halogen und/oder Heterocyclen substituiertes C₅ - C₁₂-Cycloalkyl, beispielsweise Cyclopentyl, Cyclohexyl, Cyclooctyl, Cyclododecyl, Methylcyclopentyl, Dimethylcyclopentyl, Methylcyclohexyl, Dimethylcyclohexyl, Diethylcyclohexyl, Butylcyclohexyl, Methoxycyclohexyl, Dimethoxycyclohexyl, Diethoxycyclohexyl, Butylthiocyclohexyl, Chlorcyclohexyl, Dichlorcyclohexyl, Dichlorcyclopentyl sowie ein gesättigtes oder ungesättigtes bicyclisches System wie z.B. Norbornyl oder Norbornenyl,

Heterocyclus: ein fünf- bis sechsgliedriger, Sauerstoff-, Stickstoff- und/oder Schwefelatome aufweisender Heterocyclus, beispielsweise Furyl, Thiophenyl, Pyrryl, Pyridyl, Indolyl, Benzoxazolyl, Dioxolyl, Dioxyl, Benzimidazolyl, Benzthiazolyl, Dimethylpyridyl, Methylchinolyl, Dimethylpyrryl, Methoxyfuryl, Dimethoxypyridyl, Difluorpyridyl, Methylthiophenyl, Isopropylthiophenyl oder tert.-Butylthiophenyl, gegebenenfalls kann der Heterocyclus durch funktionelle Gruppen, Aryl, Alkyl, Aryloxy, Alkyloxy, Halogen und/oder weitere Heterocyclen substituiert sein, und
C₁ bis C₄-Alkyl beispielsweise Methyl, Ethyl, Propyl, Isopropyl, n-Butyl, sec-Butyl oder tert.-Butyl.
C₁ - C₁₈-Alkyloyl (Alkylcarbonyl) kann beispielsweise sein Acetyl, Propionyl, n-Butyloyl, sec-Butyloyl, tert.-Butyloyl, 2-Etylhexylcarbonyl, Decanoyl, Dodecanoyl, Chloracetyl, Trichloracetyl oder Trifluoracetyl.
C₁ - C₁₈- Alkyloxycarbonyl kann beispielsweise sein Methyloxycarbonyl, Ethyloxycarbonyl, Propyloxycarbonyl, lsopropyloxycarbonyl, n-Butyloxycarbonyl, sec-Butyloxycarbonyl, tert.-Butyloxycarbonyl, Hexyloxycarbonyl, 2-Etylhexyloxycarbonyl oder Benzyloxycarbonyl.
C₅ - C₁₂-Cycloalkylcarbonyl kann beispielsweise sein Cyclopentylcarbonyl, Cyclohexylcarbonyl oder Cyclododecylcarbonyl.
C₆ - C₁₂-Aryloyl (Arylcarbonyl) kann beispielsweise sein Benzoyl, Toluyl, Xyloyl, α-Naphthoyl, β-Naphthoyl, Chlorbenzoyl, Dichlorbenzoyl, Trichlorbenzoyl oder Trimethylbenzoyl.

Im Falle von Alkyloxy- (Alkoxy-) und Aryloxy-Substituenten weisen deren Alkyl- beziehungsweise Aryl-Fragmente die vorstehenden Definitionen für Alkyl beziehungsweise Aryl auf.

Bevorzugt sind R¹, R², R³, und R⁴ unabhängig voneinander Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Dimethylamino, Diethylamino oder Chlor.

Bevorzugt ist R⁷ Wasserstoff, Methyl, Ethyl, n-Butyl, 2-Hydroxyethyl, 2-Cyanoethyl, 2-(Methoxycarbonyl)-ethyl, 2-(Ethoxycarbonyl)-ethyl, 2-(n-Butoxycarbonyl)-ethyl, Acetyl, Propionyl, t-Butyryl, Methoxycarbonyl, Ethoxycarbonyl oder n-Butoxycarbonyl.

Mehr bevorzugte Imidazoliumionen (e) sind solche, bei denen unabhängig voneinander
R¹ ausgewählt ist unter Methyl, Ethyl, n-Propyl, n-Butyl, n-Pentyl, n-Octyl, n-Decyl, n-Dodecyl, 2-Hydroxyethyl oder 2-Cyanoethyl,
R⁷ Wasserstoff, Acetyl, Methyl, Ethyl, n-Propyl oder n-Butyl und
R² bis R⁴ unabhängig voneinander Wasserstoff, Methyl oder Ethyl bedeuten.

Als Anionen sind prinzipiell alle Anionen denkbar.

Bevorzugt als Anionen sind Halogenide, (F, Cl⁻, Br⁻, I⁻), Alkylcarboxylat (C₁-C₁₈-Alkyl-CO₂⁻), Tosylat (p-CH₃C₆H₄SO₃⁻), Sulfonat (C₁-C₁₈-Alkyl-SO₃⁻), Dialkylphosphat (Di(C₁-C₁₈-Alkyl)-PO₄⁻), Bis(trifluormethylsulfonyl)imid ((CF₃SO₂)₂N⁻), Trifluoracetat (CF₃COO⁻), Triflat (CF₃SO₃⁻), Sulfat (SO₄²⁻) , Hydrogensulfat (HSO₄⁻), Methylsulfat (CH₃OSO₃⁻), Ethylsulfat (C₂H₅OSO₃⁻), Sulfit (SO₃²⁻), Hydrogensulfit (HSO₃⁻), Chloro-aluminate (AlCl₄⁻) (Al₂Cl₇⁻), (Al₃Cl₁₀⁻), Bromoaluminate (AlBr₄⁻), Nitrit (NO₂⁻), Nitrat (NO₃⁻), Kupferchlorid (CuCl₂⁻), Phosphat (PO₄³-), Hydrogenphosphat (HPO₄²⁻), Dihydrogenphosphat (H₂PO₄⁻), Carbonat (CO₃²⁻) und Hydrogencarbonat (HCO₃⁻).

Mehr bevorzugt als Anionen sind Halogenide, Acetat, Methansulfonat, Tosylat, Sulfat, Hydrogensulfat, Phosphat, Hydrogenphosphat, Dihydrogenphosphat, Dialkylphosphat und Bis(trifluormethylsulfonyl)imid.

Besonders bevorzugt als Anionen sind Chlorid, Bromid, Hydrogensulfat und Diethylphosphat.

Im erfindungsgemäßen Verfahren können prinzipiell alle Alkohole verwendet werden, auch solche, die über zwei oder mehr OH-Gruppen verfügen. Gegebenenfalls können die Alkohole auch einfach oder mehrfach substituiert sein.

Bevorzugte Alkohole sind: lineare, verzweigte oder cyclische C₁-C₂₀-Alkohole. Mehr bevorzugt sind lineare, verzweigte oder cyclische C₁-C₁₀-Alkohole, wie sec-Butanol, Isobutanol, 2-Ethylhexanol, 2-Propylheptanol, Isononanol, Cyclohexanol, Cyclopentanol, Glykol, 1,3-Propandiol, 1,4-Butandiol, 1,5-Pentandiol, 1,6-Hexandiol, Neopentylglycol, Trimethylolpropan, Pentaerythrit, Glycerin, Trimethylolethan, 1,2-Propandiol, 1,2-Butandiol, 2,3-Butandiol, Allylalkohol, Propargylalkohol, Diethylenglykol und Triethylenglykol.

Besonders bevorzugte Alkohole sind: 1,6-Hexandiol, 1,5 Pentandiol, 1,4-Butandiol, 1,3-Propandiol, Glykol, Allylalkohol und Propargylalkohol.

Die Halogenierung im erfindungsgemäßen Verfahren wird mit Halogenwasserstoff durchgeführt, der ausschließlich gasförmig eingesetzt wird. Als Halogenwasserstoff eignet sich Fluorwasserstoff, Chlorwasserstoff, Bromwasserstoff und Iodwasserstoff, bevorzugt Chlorwasserstoff und Bromwasserstoff, besonders bevorzugt Chlorwasserstoff.

Das erfindungsgemäße Verfahren wird gewöhnlich so durchgeführt, dass einer der vorgenannten Alkohole mit einem der vorgenannten Halogenwasserstoffe reagiert.

Gegebenfalls können aber auch Gemische von Alkoholen und/oder Halogenwasserstoffen eingesetzt werden.

Bei der Reaktion des Alkohols mit Halogenwasserstoff können im Fall von Alkoholen, die mehr als eine OH-Gruppe pro Molekül aufweisen, entweder sämtliche OH-Gruppen durch ein Halogen ersetzt werden oder die Reaktion läuft derart ab, dass nur ein Teil der OH-Gruppen des entsprechenden Alkohols (pro Molekül) - beispielsweise bei Glycerin oder 1,3-Propandiol - durch Halogen ersetzt werden. Folglich können im Fall von 1,3-Propandiol entweder 3-Chlorpropanol oder 1,3-Dichlorpropan im erfindungsgemäßen Verfahren hergestellt werden, wobei die Reaktion über die Menge an zugegebenem Halogenwasserstoff gesteuert wird. Vorzugsweise werden im erfindungsgemäßen Verfahren sämtliche OH-Gruppen eines Alkohols durch Halogen ersetzt.

Im erfindungsgemäßen Verfahren wird die ionische Flüssigkeit vorgelegt, dann wird eine Temperatur eingestellt, die über dem Schmelzpunkt der ionischen Flüssigkeit liegt. Anschließend kann Halogenwasserstoff eingeleitet werden, bis eine Sättigung der ionischen Flüssigkeit erreicht ist. Daraufhin wird der Alkohol zugegeben und im Anschluss an die Alkoholzugabe (wiederum) Halogenwasserstoff eingeleitet. Gegebenenfalls kann auch der Alkohol vorgelegt werden und die gesättigte ionische Flüssigkeit wird bei Temperaturen, die über dem Schmelzpunkt der ionischen Flüssigkeit liegen, in den Alkohol eingeleitet. Vorzugsweise wird der Alkohol in die gesättigte ionische Flüssigkeit eingeleitet.

Bevorzugt wird das erfindungsgemäße Verfahren so durchgeführt, dass für jedes Mol an umzusetzender OH-Gruppe des Alkohols die Reaktion in Gegenwart der 0,3 bis 3-fach, mehr bevorzugt 1 bis 3-fach, besonders bevorzugt 1 bis 2-fach, molaren Menge an ionischer Flüssigkeit durchgeführt wird. Gegebenenfalls kann die Reaktion auch bei einem größeren molaren Überschuss an ionischer Flüssigkeit durchgeführt werden, wobei aber aufgrund der Volumenzunahme eine Verringerung der Raum/Zeit-Ausbeute zu beobachten ist. Ebenso ist auch ein Unterschreiten der 0,3-fach molaren Mengen an ionischer Flüssigkeit denkbar.

Das erfindungsgemäße Verfahren wird zumindest zeitweise bei Temperaturen über 100 °C durchgeführt. Vorzugsweise wird die Reaktion zumindest zeitweise bei einer Temperatur zwischen 110 °C und 150 °C durchgeführt. Mehr bevorzugt sind eine zumindest zeitweise Reaktionsführung bei 120 °C bis 145 °C, besonders bevorzugt zumindest zeitweise bei 125 °C bis 140 °C.

Im erfindungsgemäßen Verfahren kann der Alkohol bei Temperaturen unter 100°C zu der ionischen Flüssigkeit zugegeben werden. Die anschließende Einleitung des gasförmigen Halogenwasserstoffs in das den Alkohol und die ionische Flüssigkeit enthaltende Gemisch kann ebenfalls - teilweise oder vollständig - bei Temperaturen unter 100°C erfolgen. Sofern die Zugabe des Alkohols in die ionische Flüssigkeit und/oder die anschließende Einleitung des gasförmigen Halogenwasserstoffs - teilweise oder vollständig - bei Temperaturen unter 100°C durchgeführt wird, erfolgt dies vorzugsweise bei mindestens 20°C, mehr bevorzugt bei mindestens 50°C, noch mehr bevorzugt bei mindestens 75°C und besonders bevorzugt bei 85°C. Voraussetzung für eine selektive Umsetzung des Alkohols zum entsprechenden Halogenalkan ist jedoch, dass nicht vor der Einleitung des gasförmigen Halogenwasserstoffs in das den Alkohol und die ionische Flüssigkeit enthaltende Gemisch, spätestens aber nach Beendigung der Einleitung des gasförmigen Halogenwasserstoffs, das dabei gebildete Gemisch enthaltend Halogenwasserstoff den Alkohol und die ionische Flüssigkeit zeitweise auf Temperaturen über 100°C aufgeheizt wird. Erst diese Erhöhung der Reaktionstemperatur auf Temperaturen über 100°C bewirkt eine vollständige und selektive Umsetzung zum gewünschten Halogenalkan.

Die Reaktionstemperatur muss für eine ausreichende Zeit auf Temperaturen über 100°C erhöht werden. Die Zeitspanne der Temperaturerhöhung auf über 100°C sollte 1 min nicht unterschreiten, vorzugsweise beträgt sie mehr als 1 min, mehr bevorzugt mehr als 5 min, viel mehr bevorzugt mehr als 15 min, besonders bevorzugt mehr als 30 min. Vorzugsweise wird die Temperatur erst zum Ende der Halogenwasserstoffzugabe in das den Alkohol enthaltende Gemisch auf über 100°C erhöht. Vorzugsweise erfolgt die Erhöhung der Reaktionstemperatur auf über 100°C in Form einer Temperaturrampe, das heißt, die Reaktionstemperatur wird nach zumindest teilweiser Halogenwasserstoffzugabe kontinuierlich auf Temperaturen über 100°C erhöht und das Reaktionsgemisch wird bis zur vollständigen Umsetzung auf über 100°C gehalten.

In einer Ausführungsform der vorliegenden Erfindung kann jedoch die gesamte Zugabe des Halogenwasserstoffs in das den Alkohol enthaltende Gemisch bei Temperaturen von über 100°C erfolgen. Gegebenenfalls kann auch die Zugabe des Alkohols in die ionische Flüssigkeit bei Temperaturen über 100°C erfolgen. Bezüglich der bevorzugten (mehr bevorzugten usw.) Temperaturbereiche gelten die gleichen Bereichsangaben wie für die Reaktionsführung mit der nur zeitweisen Temperaturerhöhung auf über 100°C.

Bevorzugt wird das erfindungsgemäße Verfahren nur zeitweise bei Temperaturen von über 100°C durchgeführt. Dadurch lassen sich noch bessere Selektivitäten hinsichtlich des darzustellenden Halogenalkans gegenüber dem als Nebenprodukt anfallenden Ether sowie ein verbesserter Umsatz erzielen.

Je geringer der Wassergehalt zu Beginn der Halogenierungsreaktion ist, umso schneller erfolgt die Umsetzung von Alkoholen mit Halogenwasserstoffen. Demzufolge darf im erfindungsgemäßen Verfahren zumindest zum Zeitpunkt des Reaktionsbeginns der Wassergehalt maximal 25 Mol% bezogen auf die Menge an ionischer Flüssigkeit betragen. Bevorzugt ist hierbei ein maximaler Wassergehalt von 20 Mol%, mehr bevorzugt maximal 10 Mol%, noch mehr bevorzugt maximal 5 Mol%, besonders bevorzugt ist, dass zum Zeitpunkt des Reaktionsbeginns die Reaktion wasserfrei oder weitgehend wasserfrei ist. Unter weitgehender Wasserfreiheit werden nachfolgend Verunreinigungen an Wasser im ppm-Bereich betrachtet.

Die mit dem erfindungsgemäßen Verfahren hergestellten Halogenalkane können durch mit dem Fachmann bekannten Verfahren nach Beendigung der Reaktion aus dem Reaktionsgemisch isoliert werden. Sofern sich im Reaktionsgemisch ein zweiphasiges System ausbildet, kann das Produkt (Halogenalkan) durch einfache Phasentrennung isoliert werden, gegebenenfalls können sich in der anderen Phase des zweiphasigen Systems befindliche Produktnebenmengen durch zusätzliche Extraktionsschritte oder Destillation aus dieser Phase isoliert werden, sofern sich ein einphasiges Reaktionsgemisch ausbildet, kann das Produkt durch Extraktion oder Destillation aus dem Reaktionsgemisch isoliert werden. Vorzugsweise wird das Produkt durch Destillation aus dem Reaktionsgemisch erhalten, insbesondere unter Verwendung einer Destillationsbrücke unter reduziertem Druck. Sofern bei dieser Destillation ein zweiphasiges Gemisch anfällt, kann das Produkt beispielsweise durch Abtrennung, Extraktion oder gegebenenfalls durch zusätzliche Destillationsschritte isoliert werden.

In einer weiteren Ausführungsform wird das erfindungsgemäße Verfahren derart durchgeführt, dass über die gesamte Reaktionszeit der Wassergehalt bei maximal 25 Mol% bezogen auf die Menge an ionischer Flüssigkeit beträgt, bevorzugt ist ein maximaler Wassergehalt von 20 Mol%, mehr bevorzugt maximal 10 Mol%, besonders bevorzugt maximal 5 Mol%. Diese Begrenzung des maximalen Wassergehalts über den gesamten Reaktionszeitraum wird dadurch erreicht, dass das bei der Reaktion des Alkohols mit Halogenwasserstoff frei werdende Reaktionswasser beziehungsweise das sich bereits vorher im System befindliche Wasser kontinuierlich aus dem System entfernt wird. Wasser kann beispielsweise durch Abdestillieren oder den Einsatz von Trockenmitteln oder Membranen aus dem System entfernt werden. In bevorzugter Weise erfolgt dies durch Abdestillieren des Wassers. Die kontinuierliche Entfernung des Reaktionswassers ist dahingehend von Vorteil, da die Umsetzung umso schneller abläuft, je weniger Wasser sich im System befindet und durch die Zunahme des Gehaltes an Reaktionswasser eine stetige Verlangsamung der Umsetzungsgeschwindigkeit und somit eine Abnahme der Raum/Zeit-Ausbeute zu beobachten ist. Das erfindungsgemäße Verfahren kann sowohl kontinuierlich als auch diskontinuierlich betrieben werden. Unter kontinuierlicher Fahrweise versteht man, dass nicht nur das Wasser kontinuierlich entfernt wird, sondern auch die Edukte und gegebenenfalls das Produkt kontinuierlich nachgeliefert beziehungsweise entfernt werden.

Die ionischen Flüssigkeiten können im erfindungsgemäßen Verfahren entweder direkt eingesetzt werden, oder sie werden unmittelbar vor der Umsetzung des Alkohols mit Halogenwasserstoff durch Einleiten in sowie Sättigen der entsprechenden Base hergestellt.

Die nachfolgenden Beispiele sollen die Erfindung näher erläutern:

### Beispiel 1

Bei einer Temperatur von ca. 93 °C wird durch Eingasen von 51,7 g Chlorwasserstoff in 82,0 g 1-Methylimidazol das unter diesen Bedingungen flüssige Hydrochlorid hergestellt und anschließend auf 135 °C aufgeheizt. Bei dieser Temperatur wird nach Zugabe von 59,0 g 1,6-Hexandiol 83,3 g Chlorwasserstoff in das Reaktionsgemisch geleitet, wobei sich ein flüssiges Zweiphasengemisch ausbildet. Der Umsatz ist vollständig. Durch Abtrennung der Oberphase wird 66,5 g (81,4%) 1,6-Dichlorhexan in einer Reinheit (GC) von 96,2% erhalten, das außerdem noch 3,2% Bis-(6-chlorhexyl)-ether enthält.
Durch Destillation der Unterphase werden weitere 3,3 g (4,1%) 1,6-Dichlorhexan in einer Reinheit von 98,2% erhalten.

### Vergleichsbeispiel 1 A

Bei einer Temperatur von 135 °C werden 82,0 g 1-Methylimidazol und 59,0 g 1,6-Hexandiol unter Rühren vorgelegt und anschließend 85,6 g Chlorwasserstoff eingeleitet, wobei sich ein flüssiges Zweiphasengemisch ausbildet. Der Umsatz liegt bei 99,9%. Durch Abtrennung der Oberphase werden 67,3 g (74,1 %) 1,6-Dichlorhexan in einer Reinheit (GC) von 88,5% erhalten, das außerdem noch 8,2% Bis-(6-chlorhexyl)-ether enthält.

### Vergleichsbeispiel 1 B

Bei einer Temperatur von ca. 86 °C wird durch Eingasen von 56,7 g Chlorwasserstoff in 82,0 g 1-Methylimidazol das unter diesen Bedingungen flüssige Hydrochlorid hergestellt. Anschließend werden 59,0 g 1,6-Hexandiol zugegeben und bei 80 bis 86 °C Chlorwasserstoff (49,3 g) eingeleitet. Die Analyse des einphasigen Reaktionsgemisches ergibt einen Umsatz von 49,7%. Der Gehalt an 1,6-Dichlorhexan entspricht einer Ausbeute von 8,7%.

Aus Beispiel 1 wird ersichtlich, dass die Umsetzung des Alkohols mit Halogenwasserstoff bei Temperaturen > 100 °C und in Gegenwart einer ionischen Flüssigkeit gegenüber dem Stand der Technik (Vergleichsbeispiele 1A, 1B) Vorteile offeriert. In Vergleichsbeispiel 1 A wird anstelle der ionischen Flüssigkeit die entsprechende freie Base verwendet. Die Gesamtausbeute am Reaktionsprodukt ist niedriger, es fällt eine höhere Menge an Ether als Nebenprodukt an. Gegenüber Vergleichsbeispiel 1 B wird die Reaktion bei höheren Temperaturen durchgeführt, dies wirkt sich positiv sowohl auf den Umsatz als auch auf die Ausbeute (höhere Selektivität) an Reaktionsprodukt (Halogenalkan) aus.

### Beispiele 2-4

In einem HWS-Rührreaktor werden 1 Mol Base beziehungsweise ionische Flüssigkeit vorgelegt. Danach wird auf 135 °C aufgeheizt. Durch Einleiten von Chlorwasserstoff bis zur Sättigung wird aus der Base die entsprechende ionische Flüssigkeit hergestellt. Im vorliegenden Fall wird auch in die ionische Flüssigkeit Chlorwasserstoff eingeleitet, um gegenüber der freien Base gleiche Ausgangsbedingungen zu haben, dies ist aber nicht unbedingt erforderlich. Anschließend wird mit 0,5 Mol 1,6-Hexandiol versetzt und danach Chlorwasserstoff eingeleitet, wobei die Aufnahme von HCl über eine Gasbürette verfolgt wird.

**Tabelle 1**

| Beispiel | Base/ionische Flüssigkeit | Wassermenge | Dauer der HCl-Aufnahme (min:sec) | | |
|---|---|---|---|---|---|
| | | | 500 ml | 1000 ml | 1500 ml |
| 2 | 1-Methylimidazol | -- | 0:28 | 1:01 | 1:54 |
| 3 | 2-Ethylpyridin (Vergleichsbeispiel) | -- | 0:25 | 0:56 | 1:42 |
| 4 | 1-Ethyl-3-methylimidazoliumchlorid | -- | 0:19 | 0:40 | 1:06 |

### Vergleichsbeispiele 2-5

Die Vergleichsbeispiele werden entsprechend den erfindungsgemäßen Beispielen ausgeführt, mit dem Unterschied, dass zusätzlich zur Base beziehungsweise ionischen Flüssigkeit die in der Tabelle angegebene Wassermenge (bezogen auf die ionische Flüssigkeit) vorgelegt wird und die Lösung anschließend mit Chlorwasserstoff gesättigt wird.

**Tabelle 2**

| Vergleichs-Beispiel | Base/ionische Flüssigkeit | Wassermenge [Mol] | Dauer der HCl-Aufnahme (min:sec) | | |
|---|---|---|---|---|---|
| | | | 500 ml | 1000 ml | 1500 ml |
| V2 | 1-Methylimidazol | 0,5 | 1:07 | 2:13 | 4:13 |
| V3 | 2-Ethylpyridin | 1 | 0:35 | 1:29 | 2:55 |
| V4A | 1-Ethyl-3-methylimidazoliumchlorid | 0,5 | 1:01 | 1:35 | 2:20 |
| V4B | 1-Ethyl-3-methylimidazoliumchlorid | 1 | 1:03 | 2:33 | 4:50 |
| V5 | Tri-n-butylamin | 1 | 0:42 | 1:53 | 4:55 |

Die Dauer der HCl-Aufnahme ist ein Maß für die Umsetzungsgeschwindigkeit. Aus den vorstehenden Tabellen wird ersichtlich, dass die Umsetzung nach dem erfindungsgemäßen Verfahren deutlich schneller erfolgt, was eine Verbesserung der Raum/Zeit-Ausbeute zur Folge hat. Bei einer HCl-Aufnahme von ca. 1500 ml ergibt sich unter Annahme einer vollständigen Umsetzung an eingeleitetem HCl sowie dessen Verhalten als ideales Gas eine freigesetzte Menge an Reaktionswasser von maximal 30 Mol%. Die Versuche zeigen weiterhin, dass durch die Verwendung von ionischen Flüssigkeiten bei höheren Temperaturen und anfänglicher Wasserfreiheit trotz zunehmender Menge an Reaktionswasser (bis maximal 30 Mol%) keine merkliche Verlangsamung der Umsetzungsgeschwindigkeit zu beobachten ist.

### Beispiel 5

Bei einer Temperatur von 135 °C wird 118,5 g (1,0 mol) 1-Methylimidazolhydrochlorid als Schmelze vorgelegt und mit 59,0 g (0,5 mol) festem 1,6-Hexandiol versetzt. Anschließend wird 47,3 g (1,29 mol) Chlorwasserstoffgas innerhalb 35 Minuten als gleichmäßiger Gasstrom in die Reaktionsmischung geleitet. Anschließend wird über eine Destillationsbrücke unter reduziertem Druck so lange destlilliert bis bei einer Innentemperatur von 139 °C und 33 mbar kein Produkt mehr übergeht. Das Destillat fällt als zweiphasiges Gemisch an. Nach Abtrennung der wässrigen Phase wird 69,7 g (88,8 %) 1,6-Dichlorhexan in einer Reinheit (GC) von 98,7% erhalten.

### Beispiel 6 (Vergleichsbeispiel)

Bei einer Temperatur von 85 °C wird durch Eingasen von 44,6 g (1,22 mol) Chlorwasserstoff in 82,0 g (1,0 mol) 1-Methylimidazol das unter diesen Bedingungen flüssige Hydrochlorid hergestellt, mit 18,0 g (1,0 mol) Wasser versetzt und anschließend auf 135 °C aufgeheizt. Bei dieser Temperatur wird nach Zugabe von 59,0 g (0,5 mol) 1,6-Hexandiol innerhalb von 4 h 67,7 g (1,85 mol) Chlorwasserstoffgas in das Reaktionsgemisch geleitet, wobei sich ein Zweiphasengemisch ausbildet. Anschließend wird über eine Destillationsbrücke unter reduziertem Druck so lange destlilliert bis bei einer Innentemperatur von 135 °C und 39 mbar kein Produkt mehr übergeht. Durch Trennung des zweiphasigen Destillats wird 58,1 g (74,0%) 1,6-Dichlorhexan in einer Reinheit (GC) von 98,7 % erhalten.

### Beispiel 7

Bei einer Temperatur von bis zu 111 °C werden 82,0 g (1,0 mol) 1-Methylimidazol durch Eingasen von 45,3 g (1,24 mol) Chlorwasserstoff in 1-Methylimidazolhydrochlorid überführt und bei 98 °C mit 59,0 g (0,5 mol) festem 1,6-Hexandiol versetzt. Anschließend wird innerhalb 2,5 h 37,8 g (1,04 mol) Chlorwasserstoffgas in einem gleichmäßigen Gasstrom in die Reaktionsmischung geleitet. Dabei werden in den ersten 130 Minuten die Temperatur auf 83 bis 88 °C eingeregelt, dann wird innerhalb 20 Minuten auf 135 °C aufgeheizt, die HCl-Einleitung beendet und zwei Stunden nachgerührt. Danach wird über eine Destillationsbrücke unter reduziertem Druck so lange destlilliert bis bei einer Innentemperatur von 140 °C und 24 mbar kein Produkt mehr übergeht. Das Destillat fällt als zweiphasiges Gemisch an. Nach Abtrennung der wässrigen Phase wird 72,7 g (95,5 %) 1,6-Dichlorhexan in einer Reinheit (GC) von 99,1% erhalten.

Beim Vergleich des Beispieles 7 mit Beispiel 5 wird ersichtlich, dass bei der Verwendung einer Temperaturrampe, d.h., dass die Umsetzung von Alkohol mit Halogenwasserstoff in Gegenwart einer ionischen Flüssigkeit nur zeitweise bei Temperaturen von über 100°C durchgeführt wird, zu einer verbesserten Selektivität sowie einem verbesserten Umsatz führt. Weiterhin zeigt auch der Vergleich mit Beispiel 6, dass die Verwendung von ionischen Flüssigkeiten in wässriger Phase zu einem deutlich verminderten Umsatz des gewünschten Produkts (hier 1,6-Dichlorhexan) führt.

## Patentansprüche

1. Verfahren zur Herstellung von Halogenalkanen durch Umsetzung von Alkohol mit Halogenwasserstoff, **dadurch gekennzeichnet, dass** die Reaktion des Alkohols mit dem Halogenwasserstoff in Gegenwart einer ionischen Flüssigkeit zumindest zeitweise bei einer Temperatur von über 100°C durchgeführt wird und zumindest zum Zeitpunkt des Reaktionsbeginns der Wassergehalt maximal 25 Mol% bezogen auf die Menge an ionischer Flüssigkeit beträgt, wobei die ionische Flüssigkeit nicht Octyltrimethylammoniumchlorid ist und
das Kation der ionischen Flüssigkeit ein Imidazoliumion der Formel (e) 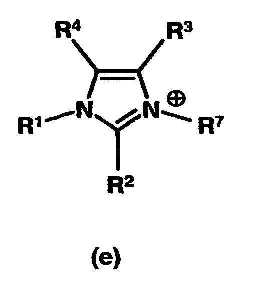 ist, worin R¹, R², R³, R⁴ und R⁷ unabhängig voneinander jeweils Wasserstoff, C₁-C₁₈-Alkyl, gegebenenfalls durch ein oder mehrere Sauerstoff- und/oder Schwefelatome und/oder ein oder mehrere substituierte oder unsubstituierte Iminogruppen unterbrochenes C₂-C₁₈-Alkyl, C₆-C₁₂-Aryl, C₅-C₁₂-Cycloalkyl oder einen fünf- bis sechsgliedrigen, Sauerstoff-, Stickstoff- und/oder Schwefelatome aufweisenden Heterocyclus bedeuten oder zwei von ihnen gemeinsam einen ungesättigten, gesättigten oder aromatischen und gegebenenfalls durch ein oder mehrere Sauerstoff- und/oder Schwefelatome und/oder ein oder mehrere substituierte oder unsubstituierte Iminogruppen unterbrochenen Ring bilden, wobei die genannten Reste jeweils durch funktionelle Gruppen, Aryl, Alkyl, Aryloxy, Alkyloxy, Halogen und/oder Heterocyclen substituiert sein können, und
R⁷ kann darüber hinaus C₁-C₁₈-Alkyloyl (Alkylcarbonyl), C₁-C₁₈-Alkyloxycarbonyl, C₅-C₁₂-Cycloalkylcarbonyl oder C₆-C₁₂-Aryloyl (Arylcarbonyl) bedeuten, wobei die genannten Reste jeweils durch funktionelle Gruppen, Aryl, Alkyl, Aryloxy, Alkyloxy, Halogen und/oder Heterocyclen substituiert sein können,
wobei funktionelle Gruppen Carboxy, Carboxamid, Hydroxy, Amino, C₁-C₄-Alkylamino, Di-(C₁-C₄-alkyl)-amino, C₁-C₄- Alkyloxy carbonyl, Cyano oder C₁-C₄-Alkyloxy bedeuten.

2. Verfahren nach Anspruch 1, **dadurch gekennzeichnet, dass** der Halogenwasserstoff teilweise oder vollständig bei Temperaturen unter 100°C in das den Alkohol und die ionische Flüssigkeit enthaltende Gemisch eingeleitet wird und das dabei gebildete Gemisch zeitweise auf Temperaturen über 100°C aufgeheizt wird.

3. Verfahren nach Anspruch 1 oder 2, **dadurch gekennzeichnet, dass** die Reaktion bei einer Temperatur zwischen 110°C und 150°C durchgeführt wird.

4. Verfahren nach einem der Ansprüche 1 bis 3, **dadurch gekennzeichnet, dass** als Halogenwasserstoff HCI oder HBr verwendet wird.

5. Verfahren nach einem der Ansprüche 1 bis 4, **dadurch gekennzeichnet, dass** ein Alkohol ausgewählt aus der Gruppe bestehend aus sec-Butanol, Isobutanol, 2-Ethylhexanol, 2-Propylheptanol, Isononanol, Cyclohexanol, Cyclopentanol, Glykol, 1,3-Propandiol, 1,4-Butandiol, 1,5-Pentandiol, 1,6-Hexandiol, Neopentylglycol, Trimethylolpropan, Pentaerythrit, Glycerin, Trimethylolethan, 1,2-Propandiol, 1,2-Butandiol, 2,3-Butandiol, Allylalkohol, Propargylalkohol, Diethylenglykol und Triethylenglykol, insbesondere ausgewählt aus der Gruppe bestehend aus 1,6-Hexandiol, 1,5-Pentandiol, 1,4-Butandiol, 1,3-Propandiol, Glykol, Allylalkohol und Propargylalkohol, verwendet wird.

6. Verfahren nach einem der Ansprüche 1 bis 5, **dadurch gekennzeichnet, dass** eine ionische Flüssigkeit verwendet wird,
wobei das Anion ausgewählt ist aus der Gruppe bestehend aus:
Halogenide, Alkylcarboxylat, Tosylat, Sulfonat, Dialkylphosphat, Bis(trifluormethylsulfonyl)imid, Trifluoracetat, Triflat, Sulfat, Hydrogensulfat, Methylsulfat, Ethylsulfat, Sulfit, Hydrogensulfit, Chloro-aluminate, Bromoaluminate, Nitrit, Nitrat, Kupferchlorid, Phosphat, Hydrogenphosphat, Dihydrogenphosphat, Carbonat und Hydrogencarbonat.

7. Verfahren nach einem der Ansprüche 1 bis 6, **dadurch gekennzeichnet, dass** das Halogenalkan durch Destillation aus dem Reaktionsgemisch isoliert wird.

8. Verfahren nach einem der Ansprüche 1 bis 7, **dadurch gekennzeichnet, dass** die ionische Flüssigkeit einen Schmelzpunkt von unter 150°C, insbesondere unter 100°C, aufweist.

9. Verfahren nach einem der Ansprüche 1 bis 8, **dadurch gekennzeichnet, dass** für jedes Mol an umzusetzender OH-Gruppe des Alkohols die Reaktion in Gegenwart der 1- bis 3-fachen molaren Menge an ionischer Flüssigkeit durchgeführt wird.

10. Verfahren nach einem der Ansprüche 1 bis 9, **dadurch gekennzeichnet, dass** zum Zeitpunkt des Reaktionsbeginns die Reaktion wasserfrei oder weitgehend wasserfrei durchgeführt wird.

11. Verfahren nach einem der Ansprüche 1 bis 10, **dadurch gekennzeichnet, dass** das bei der Reaktion freiwerdende Wasser kontinuierlich entfernt, insbesondere abdestilliert wird.

12. Verfahren nach Anspruch 11, **dadurch gekennzeichnet, dass** der Wassergehalt bei der Reaktion des Alkohols mit dem Halogenwasserstoff über die gesamte Reaktionszeit maximal 25 Mol%, bevorzugt maximal 20 Mol%, mehr bevorzugt maximal 10 Mol%, besonders bevorzugt maximal 5 Mol%, bezogen auf die Menge an ionischer Flüssigkeit beträgt.

13. Verfahren nach einem der Ansprüche 1 bis 12, **dadurch gekennzeichnet, dass** bei der Umsetzung von Alkoholen, die mehr als eine OH-Gruppe pro Molekül aufweisen, sämtliche OH-Gruppen durch Halogen ersetzt werden.

## Claims

1. A process for preparing haloalkanes by reaction of alcohol with hydrogen halide, wherein the reaction of the alcohol with the hydrogen halide occurs in the presence of an ionic liquid for at least part of the time at a temperature above 100°C and, at least at the time of commencement of the reaction, the water content is not more than 25 mol% based on the amount of ionic liquid, where the ionic liquid is not octyltrimethylammonium chloride and the cation of the ionic liquid is an imidazolium ion of the formula (e) 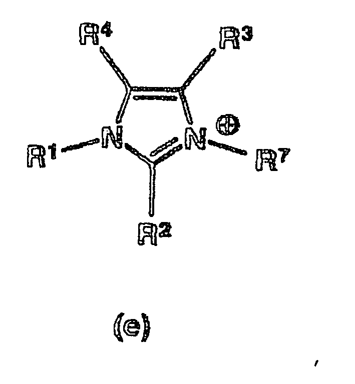 where
R¹, R², R³, R⁴ and R⁷ are each, independently of one another, hydrogen, C₁-C₁₈-alkyl, C₂-C₁₈-alkyl which may be interrupted by one or_more oxygen and/or sulfur atoms and/or one or more substituted or unsubstituted imino groups, C₆-C₁₂-aryl, C₅-C₁₂-cycloalkyl or a five- or six-membered, oxygen-, nitrogen- and/or sulfur-containing heterocycle or two of them together form an unsaturated, saturated or aromatic ring which may be interrupted by one or more oxygen and/or sulfur atoms and/or one or more substituted or unsubstituted imino groups, where the radicals mentioned may each be substituted by functional groups, aryl, alkyl, aryloxy, alkyloxy, halogen and/or heterocycles, and
R⁷ can also be C₁-C₁₈-alkyloyl (alkylcarbonyl), C₁-C₁₈-alkyloxycarbonyl, C₅-C₁₂-cycloalkylcarbonyl or C₆-C₁₂-aryloyl (arylcarbonyl), where the radicals mentioned may each be substituted by functional groups, aryl, alkyl, aryloxy, alkyloxy, halogen and/or heterocycles and
functional groups are carboxyl, carboxamide, hydroxyl, amino, C₁-C₄-alkylamino, di(C₁-C₄-alkyl)-amino, C₁-C₄-alkyloxycarbonyl, cyano or C₁-C₄-alkyloxy.

2. The process according to claim 1, wherein the hydrogen halide is passed partly or entirely at temperatures below 100°C into the mixture comprising the alcohol and the ionic liquid and the mixture formed is heated for part of the time to temperatures above 100°C.

3. The process according to claim 1 or 2, wherein the reaction is carried out at from 110°C to 150°C.

4. The process according to any of claims 1 to 3, wherein HCI or HBr is used as hydrogen halide.

5. The process according to any of claims 1 to 4, wherein an alcohol selected from the group consisting of sec-butanol, isobutanol, 2-ethylhexanol, 2-propylheptanol, isononanol, cyclohexanol, cyclopentanol, glycol, 1,3-propanediol, 1,4-butanediol, 1,5-pentanediol, 1,6-hexanediol, neopentyl glycol, trimethylolpropane, pentaerythritol, glycerol, trimethylolethane, 1,2-propanediol, 1,2-butanediol, 2,3-butanediol, allyl alcohol, propargyl alcohol, diethylene glycol and triethylene glycol, in particular selected from the group consisting of 1,6-hexanediol, 1,5-pentanediol, 1,4-butanediol, 1,3-propanediol, glycol, allyl alcohol and propargyl alcohol, is used.

6. The process according to any of claims 1 to 5, wherein an ionic liquid is used and the anion is selected from the group consisting of:
Halides, alkylcarboxylate, tosylate, sulfonate, dialkylphosphate, bis(trifluoromethylsulfonylimide, trifluoracetate, triflate, sulfate, hydrogen-sulfate, methylsulfate, ethylsulfate, sulfite, hydrogensulfite, chloroaluminates, bromoaluminates, nitrite, nitrate, chlorocuprate, phosphate, hydrogenphosphate, dihydrogenphosphate, carbonate and hydrogencarbonate.

7. The process according to any of claims 1 to 6, wherein the haloalkane is isolated from the reaction mixture by distillation.

8. The process according to any of claims 1 to 7, wherein the ionic liquid has a melting point of less than 150°C, in particular less than 100°C.

9. The process according to any of claims 1 to 8, wherein the reaction is carried out in the presence of from 1 to 3 mol of ionic liquid per mol of OH group to be reacted in the alcohol.

10. The process according to any of claims 1 to 9, wherein the reaction is carried out in the absence of water or the substantial absence of water at the time of commencement of the reaction.

11. The process according to any of claims 1 to 10, wherein the water liberated in the reaction is continuously removed, in particular distilled off.

12. The process according to claim 11, wherein the water content in the reaction of the alcohol with the hydrogen halide is not more than 25 mol%, preferably not more than 20 mol%, more preferably not more than 10 mol%, particularly preferably not more than 5 mol%, based on the amount of ionic liquid, over the entire reaction time.

13. The process according to any of claims 1 to 12, wherein in the case of the reaction of alcohols having more than one OH group per molecule, all OH groups are replaced by halogen.

## Revendications

1. Procédé de préparation d'halogénoalcanes par réaction d'alcool avec un hydracide halogéné, **caractérisé en ce que** la réaction de l'alcool avec l'hydracide halogéné est effectuée en présence d'un liquide ionique au moins temporairement à des températures supérieures à 100°C et **en ce qu'**au moins au moment du commencement de la réaction la teneur en eau est au maximum de 25 mol % par rapport à la quantité de liquide ionique, le liquide ionique n'étant pas du chlorure d'octyltriméthylammonium, et
le cation du liquide ionique est un ion d'imidazolium de formule (e) 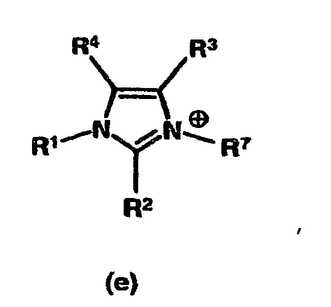
dans laquelle R¹, R², R³, R⁴ et R⁷ représentent chacun indépendamment l'un de l'autre de l'hydrogène ou un groupe alkyle en C₁-C₁₈; un groupe alkyle en C₂-C₁₈, aryle en C₆-C₁₂, cycloalkyle en C₅-C₁₂ éventuellement interrompu par un ou plusieurs atomes d'oxygène et/ou de soufre et/ou un ou plusieurs groupes imino substitués ou non substitués, ou un hétérocycle pentagonal à hexagonal, présentant des atomes d'oxygène, d'azote et/ou de soufre, ou deux d'entre eux forment conjointement un noyau non saturé, saturé ou aromatique et éventuellement interrompu par un ou plusieurs atomes d'oxygène et/ou de soufre et/ou un ou plusieurs groupes imino substitués ou non substitués, les radicaux cités pouvant chacun être substitués par des groupes fonctionnels, aryle, alkyle, aryloxy, alkyloxy, halogène et/ou des hétérocycles, et
R⁷ peut en outre représenter un groupe C₁-C₁₈-alkyloyle (alkylcarbonyle), C₁-C₁₈-alkyloxycarbonyle, C₅-C₁₂-cycloalkylcarbonyle ou C₆-C₁₂-aryloyle (arylcarbonyle), les radicaux cités pouvant chacun être substitués par des groupes fonctionnels, aryle, alkyle, aryloxy, alkyloxy, halogène et/ou des hétérocycles,
les groupes fonctionnels étant des groupes carboxy, carboxamide, hydroxy, amino, C₁-C₄-alkylamino, di- (C₁-C₄-alkyl) -amino, C₁-C₄-alkyloxycarbonyle, cyano ou C₁-C₄-alkyloxy.

2. Procédé suivant la revendication 1, **caractérisé en ce que** l'hydracide halogéné est introduit partiellement ou totalement dans le mélange contenant l'alcool et le liquide ionique à des températures inférieures à 100°C et le mélange ainsi formé est chauffé temporairement à des températures supérieures à 100°C.

3. Procédé suivant la revendication 1 ou 2, **caractérisé en ce que** la réaction est effectuée à une température comprise entre 110°C et 150°C.

4. Procédé suivant l'une des revendications 1 à 3, **caractérisé en ce que,** comme hydracide halogéné, on utilise HCI ou HBr.

5. Procédé suivant l'une des revendications 1 à 4, **caractérisé en ce qu'**on utilise un alcool choisi parmi le groupe constitué de sec-butanol, d'isobutanol, de 2-éthylhexanol, de 2-propylheptanol, d'isononanol, de cyclohexanol, de cyclopentanol, de glycol, de 1,3-propanediol, de 1,4-butanediol, de 1,5-pentanediol, de 1,6-hexanediol, de néopentylglycol, de triméthylolpropane, de pentaérythrite, de glycérine, de triméthyloléthane, de 1,2-propanediol, de 1,2-butanediol, de 2,3-butanediol, d'alcool allylique, d'alcool propargylique, de diéthylèneglycol et de triéthylèneglycol, en particulier choisi parmi le groupe constitué de 1,6-hexanediol, de 1,5-pentanediol, de 1,4-butanediol, de 1,3-propanediol, de glycol, d'alcool allylique et d'alcool propargylique.

6. Procédé suivant l'une des revendications 1 à 5, **caractérisé en ce qu'**on utilise un liquide ionique,
l'anion étant choisi parmi le groupe constitué :
des halogénures, du carboxylate d'alkyle, du tosylate, du sulfonate, du phosphate de dialkyle, du bis(trifluorométhylsulfonyl)imide, du trifluoroacétate, du triflate, du sulfate, de l'hydrogénosulfate, du méthylsulfate, de l'éthylsulfate, du sulfite, de l'hydrogénosulfite, des chloroaluminates, des bromoaluminates, du nitrite, du nitrate, du chlorure de cuivre, du phosphate, de l'hydrogénophosphate, du dihydrogénophosphate, du carbonate et de l'hydrogénocarbonate.

7. Procédé suivant l'une des revendications 1 à 6, **caractérisé en ce que** l'halogénoalcane est isolé du mélange réactionnel par distillation.

8. Procédé suivant l'une des revendications 1 à 7, **caractérisé en ce que** le liquide ionique présente un point de fusion inférieur à 150°C, en particulier inférieur à 100°C.

9. Procédé suivant l'une des revendications 1 à 8, **caractérisé en ce que,** pour chaque mole de groupe OH de l'alcool à faire réagir, la réaction est effectuée en présence d'une quantité 1 à 3 fois molaire de liquide ionique.

10. Procédé suivant l'une des revendications 1 à 9, **caractérisé en ce qu'**au moment du commencement de la réaction, celle-ci est effectuée sans eau ou largement sans eau.

11. Procédé suivant l'une des revendications 1 à 10, **caractérisé en ce que** l'eau libérée au cours de la réaction est éliminée en continu, en particulier par distillation.

12. Procédé suivant la revendication 11, **caractérisé en ce que** la teneur en eau au cours de la réaction de l'alcool avec l'hydracide halogéné est, pendant la totalité de la durée de la réaction, au maximum de 25 mol %, de préférence au maximum de 20 % molaires, plus avantageusement au maximum de 10 % molaires, particulièrement avantageusement au maximum de 5 % molaires, par rapport à la quantité de liquide ionique.

13. Procédé suivant l'une des revendications 1 à 12, **caractérisé en ce que,** lors de la réaction d'alcools qui présentent plus d'un groupe OH par molécule, l'ensemble des groupes OH est remplacé par de l'halogène.
